# EUROPEAN PATENT SPECIFICATION

(11) **EP 1 878 391 B1**
(45) Date of publication and mention of the grant of the patent: **25.03.2009**
(21) Application number: 07252537.1
(22) Date of filing: 22.06.2007
(51) Int. Cl.: A61B 17/064

(54) **Novel skin staples**
Neue Hautklammern
Nouvelles agrafes pour peau

(30) Priority: 11.07.2006 US 819965 P
(43) Date of publication of application: 16.01.2008
(60) Divisional application: 09001906.8
(73) Proprietor: Tyco Healthcare Group LP, Norwalk, Connecticut 06856 (US)
(72) Inventor: Prommersberger, Megan, Wallingford, CT 06492 (US)
(74) Representative: Alcock, David

(56) References cited:
- WO-A-20/04052594
- WO-A-20/04105621
- US-A- 4 719 917
- US-B1- 6 626 916

## Description

### CROSS-REFERENCE TO RELATED APPLICATIONS

This application claims the benefit of and priority to U.S. Provisional Patent Application No. 60/819,965 filed July 11, 2006, the entire disclosure of which is incorporated by reference wherein.

### TECHNICAL FIELD

The present disclosure provides a surgical skin staple. The staple may be constructed of a composite including a metal and a bioabsorbable shape memory polymer. Methods of stapling tissue with a staple of the present disclosure are also provided.

### BACKGROUND

Surgical staples are used to close wounds, both internally and in the skin. Some staples are metallic, including, for example, the staples disclosed in U.S. Patent No. 4,321,002. Other staples may be made of polymeric materials, including nonabsorbable materials or the bioabsorbable polymers of U.S. Patent No. 6,235,869. Staples constructed of both nonabsorbable and absorbable materials are also known; see, for example, U.S. Patent No. 4,719,917 which is considered to represent the closest prior art. Staples may be utilized in conjunction with other closure methods, including sutures and adhesives, or the staples may be utilized by themselves to close wounds in tissue, including skin.

In order to close, staples used for stapling skin may only-be acted upon by forces from one side of the stapled medium, whereas other types of staples may be acted upon by forces both above and below the stapled medium. Thus, one difficulty in the use of skin staples is the ability of the staple to sufficiently grab the tissue to which it is applied, as well as the ability of the staple to remain affixed thereto during healing. Staples having excellent penetration characteristics and the ability to remain affixed to the tissue to which they are applied during healing, especially in the case of skin staples, remain desirable.

### SUMMARY

The present invention is a surgical staple as in appended claim 1. Advantageous embodiments are defined by the dependent claims.

Methods for treating wounds with staples of the present disclosure are also disclosed. Such methods include providing a staple of the present disclosure possessing leg portions having a straight, temporary shape and applying the staple to tissue adjacent to a wound, and recovering a permanent shape of the legs which affixes the staple to tissue adjacent the wound. The leg portions of the staple is heated to recover a permanent, bent shape which affixes the-staple to tissue adjacent the wound. In some embodiments, body heat may be sufficient to recover the permanent shape of the legs.

### BRIEF DESCRIPTION OF THE DRAWINGS

FIG. 1 is a depiction of a staple of the present disclosure having a linear backspan and bioabsorbable shape memory polymeric legs attached thereto;
FIG. 2 is a depiction of a staple of the present disclosure having a backspan which is curved at its ends thereby forming the upper portion of the legs of the staple, with bioabsorbable shape memory polymeric materials forming the remainder of the legs of the staple;
FIG. 3 is a depiction of another staple of the present disclosure having a linear backspan and bioabsorbable shape memory polymeric legs attached thereto;
FIG. 4 is a depiction of a staple-of the present disclosure in its memorized shape; and
FIG. 5 is a depiction of a staple of the present disclosure in-its temporary shape.

### EMBODIMENTS

In accordance with the present disclosure, staples are provided having an excellent ability to remain affixed to tissue for the duration of healing. In embodiments, staples of the present disclosure include skin staples. The staples remain affixed to the tissue to which they are applied for an adequate period of time to permit healing. Being partly constructed of bioabsorbable materials, difficulties in the removal of the present staples (including the possibility of damaging tissue on removal), are avoided.

Briefly, the present disclosure provides-a novel composite staple including a non-absorbable portion and a portion formed from a shape memory polymeric material. The backspan of the staple, which may also be referred to herein as the crown, may be made of any biocompatible non-absorbable rigid or semi-rigid material, in embodiments a metal, while the legs are made at least in part of a bioabsorbable shape memory material. In use, the legs of a staple of the present disclosure are temporarily deformed to make insertion into tissue easier. A memorized bent or curved shape of the legs may then be recovered after insertion to enhance the ability of the staple to grab tissue. As the legs are at least partially bioabsorbable, the non-absorbable backspan may be removed or may fall off after sufficient healing without any further damage to the tissue.

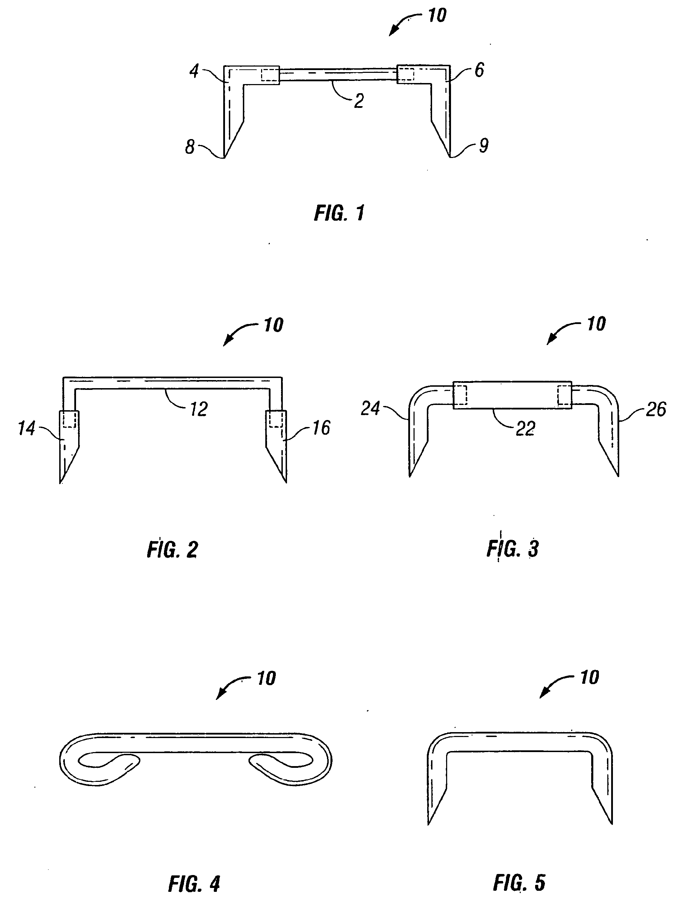

Referring now to the drawings, FIG. 1 shows-one embodiment of a staple 10 of the present disclosure. Central backspan portion 2 is linear and is attached to shape memory bioabsorbable legs 4 and 6 having tips 8 and 9. Once deformed to their temporary straight shape, the legs attached to the backspan may project perpendicularly from the backspan forming a U-shape.

FIG. 2 depicts another configuration of a staple 10 of the present disclosure, wherein central backspan portion 12 is curved at its ends, with the curved portions forming the upper sections of the legs 14 and 16 of the staple. The backspan portion 12 is of a non-absorbable material such as a metal of solid or hollow stock and the remainder of legs 14 and 16 may be made of a bioabsorbable shape memory material.

FIG. 3 shows another embodiment of a staple 10 of the present disclosure. Central backspan portion 22 is linear and is attached to shape memory bioabsorbable legs 24-and 26. While staple 10 in FIG. 1 has central backspan portion 2 embedded in shape memory bioabsorbable legs 4 and 6, the staple 10 of FIG. 3 has shape memory bioabsorbable legs 24 and 26 embedded in central backspan portion 22.

Suitable metals which may-be utilized-to form the backspan of the staple include, for example, titanium, stainless steel, steel alloys, titanium alloys, nickel chromium alloy, nickel/cobalt/chrome, and combinations thereof, although other metals, plastics, or ceramics can be used. In embodiments, titanium such as commercially pure titanium, sometimes referred to herein as CP titanium, may be utilized. CP titanium includes unalloyed titanium and may designate several grades of titanium possessing minor amounts of impurities, such as carbon, iron, and oxygen. The microstructure of CP titanium may be primarily a hexagonal-close-packed crystal structure with a relatively low strength and high ductility. The amount of oxygen may be controlled to provide increased strength. The general chemical composition (wt%) and minimum mechanical properties of the grades of CP titanium are set forth below in Table 1.

**TABLE 1**

| | Grade | | | |
|---|---|---|---|---|
| | 1 | 2 | 3 | 4 |
| Nitrogen, max. | 0.03 | 0.03 | 0.05 | 0.05 |
| Carbon, max. | 0.10 | 0.10 | 0.10 | 0.10 |
| Hydrogen, max. | 0.01 | 0.01 | 0.01 | 0.01 |
| Iron, max. | 0.20 | 0.30 | 0.30 | 0.50 |
| Oxygen, max. | 0.18 | 0.25 | 0.35 | 0.40 |
| Titanium | bal. | bal. | bal. | bal. |
| Yield strength (MPa) | 170 | 275 | 380 | 485 |
| Ultimate strength (MPa) | 240 | 345 | 450 | 550 |
| Elongation (%) | 24 | 20 | 18 | 15 |

In embodiments the backspan may be linear. In other embodiments, the backspan may be curved at its ends, thereby forming upper portions of the legs of a staple of the present disclosure, with the shape memory polymeric materials forming the lower legs of the staple of the present disclosure.

The legs of the staple are formed of bioabsorbable shape memory polymeric materials which may be resorbed in the body. Such materials include, for example, caprolactones, dioxanones, diol esters including oligo (epsilon caprolactone) diol, lactic acid, lactide, glycolic acid, glycolide, ether-ester diols including oligo (p-dioxanone) diol, carbonates including trimethylene carbonate, combinations thereof, and the like. In embodiments, the bioabsorbable shape memory polymer may be a copolymer of two components with different thermal characteristics, such as a diol ester including oligo (epsilon-caprolactone diol) and an ether-ester diol such as a crystallizable oligo (p-dioxanone) diol. These multi-block oligo (epsilon-caprolactone) diol/oligo (p-dioxanone) diol copolymers possess two block segments: -a "hard" segment and a "switching" segment linked together in linear chains. Such materials are disclosed, for example, in Lendlein, "Shape Memory Polymers-Biodegradable Sutures," Materials World, Vol. 10, no. 7, pp. 29-30 (July 2002). In other embodiments, blends of bioabsorbable materials may be utilized including, but not limited to, urethanes blended with lactic acid and/or glycolic acid, homopolymers thereof or copolymers thereof, and acrylates blended with caprolactones such as polycaprolactone dimethacrylate poly(butyl acrylate) blends, and combinations thereof.

The shape-memory polymeric materials of the present disclosure may, in embodiments, be stretched or compressed into temporary forms up to about four times larger or four times smaller than their permanent shape. In other embodiments, shape polymeric materials may be fashioned into legs of a staple of the present disclosure having a permanent shape that is curved or bent, and a temporary shape that is straight.

In embodiments, a molding process may be utilized to produce legs with the permanent shape to be memorized, for example, a bent or curved shape for holding tissue. Plastic molding methods are within the purview of those skilled in the art and include, but are not limited to, melt molding, solution molding and the like. Injection molding, extrusion molding, compression molding and other methods can also be used as the melt molding technique. Once placed in the mold with the proper bent configuration, the legs may be heated to a suitable temperature, in embodiments at a temperature referred to as the permanent temperature (Tₚₑᵣₘ), for example, from about 40° C to about 60° C, in embodiments from about 45° C to-about 55° C, for a period of time from about thirty seconds to about sixty minutes, in embodiments from about 1 minute to about 10 minutes, to obtain their permanent curved or bent shape.

After the molded legs with the desired permanent shape are formed, the legs are deformed at a deforming temperature to obtain a temporary shape. Suitable temperatures for deformation may vary depending on the shape memory polymer utilized, but generally may be above the transition temperature of the polymer (Tₜᵣₐₙₛ), but below the Tₚₑᵣₘ. The shape memory polymer is cooled from its Tₚₑᵣₘ to a lower temperature which remains above the Tₜᵣₐₙₛ and deformed to the temporary shape. In embodiments, the legs may be=straightened and cooled to room temperature (about 20° C to about 25° C) to obtain their temporary straight shape, although the temperature may differ depending upon the particular polymer employed. The legs may then be cooled to a temperature below Tₜᵣₐₙₛ, at which time the staple of the present disclosure may be ready for use.

The legs thus prepared recover their originally memorized bent or curved shapes on heating, either by placement in a patient's body or the addition of exogenous heat at a prescribed temperature, in embodiments above Tₜᵣₐₙₛ for the shape memory polymer utilized.

In other embodiments, the legs may be deformed to a straight shape and heated to assist in their adoption of their temporary shape. The temperature for deformation treatment of legs molded with a previously memorized shape is one that makes Possible ready deformation without producing cracks and should not exceed the temperature adopted for the shape memorization. Deformation treatment at a temperature exceeding that for the original shape memorization may cause the object to memorize a new deformed shape. Deformation by heating can be accomplished either by means of heaters of a special design or under an atmosphere of a suitable hot gas or liquid.

In other embodiments, legs utilized as part of a staple of the present disclosure may be formed by cold drawing, i.e., drawing a sample at a low temperature. Cold drawing methods are within the purview of those skilled in the art. Examples of these shape memory polymers and means for forming permanent and temporary shapes therewith are set forth in Lendlein et al., "Shape memory polymers as stimuli-sensitive implant materials," Clinical Hemorheology and Microcirculation, 32 (2005) 105-116, and Lendlein et al., "Biodegradable, Elastic Shape Memory Polymers for Potential Biomedical Applications," Science, Vol. 269 (2002) 1673-1676.

There are no particular limitations on the manner in-which-the-deformation can be achieved. Deformation can be achieved either by hand or by means of a suitable device selected according to the shape, size, thickness and other desirable characteristics of the molded legs.

In order to keep the shapes of the legs fixed in their temporary shape, the shape-memory molded legs of this invention should be stored at a temperature which will not cause plastic deformation of the polymers. The shape-molded memory legs may be stored in a refrigerator.

As staples of the present disclosure are utilized in a living body, heating with body heat is possible. In such a case, the temperature for shape memorization should be as low as possible and the recovery of the memorized shape may occur fairly slowly.

However, in some embodiments a higher shape-memory temperature may be desirable in order to make the shape recover at a slightly higher temperature than body temperature. Thus, in some cases releasing the molded legs from deformation to recover the originally-memorized bent or curved shape can be achieved by heating. On heating at a temperature from about 30° C to about 50° C, in embodiments from about 39° C to about 43° C, the temporary shape may be released and the memorized-permanent shape recovered. The higher the temperature for heating, the shorter the time for recovery of the originally memorized shape. The means for this heating is not limited. Like the heating for deformation treatment, heating can be accomplished by using a gas or liquid heating medium, heating devices, ultrasonic waves, or the like. Of course, in an application involving a living body, care should be taken to utilize a heating temperature which will not cause burns. When a liquid heating medium is used, physiological saline solution or alcohol-may be desirable.

The leg portions of the staple frank the central backspan portion and possess points at their ends. In embodiments, the legs of the skin staple of the present disclosure may possess sharp, penetrating tips which degrade after implantation to an extent whereby the backspan may be painlessly removed. The degradation time of the legs can be varied, for example, by the selection of the shape memory material utilized to form the leg, the coupling means and configuration utilized to attach the legs to the backspan, and the like. Times for absorption can range from about 5 days to about 2 years, in embodiments from about 1 week to about 3 months for skin staples, depending on the composition of the leg portions of the staples and their shapes.

The backspan of the staple may be physically or chemically coupled to the legs of the staple utilizing methods within the purview of one skilled in the art.

Physical coupling of the backspan portion and the leg portion can be, for example, by use of a socket in the backspan portion that frictionally or telescopically receives the leg portion, or it can be a tongue and groove frictional coupling means, or any combination of any of these coupling means. In embodiments, additional fastening means may be utilized-to mechanically attach the backspan portion to the legs. Other locking mechanisms include a locking taper, a screw, a rivet, etc. In other embodiments, combinations of the above methods may be used to physically connect the backspan to the legs. For example, the coupling means can be a mechanical fastener (e.g., tongue and groove) in combination with, for example, a bioabsorbable pin or rivet.

Chemical means for joining the backspan to the legs may include, for example, injection molding the shape memory legs onto the ends of the backspan portion of the staple. In an injection molding process, the leg portion of a staple may be adhesively coupled to the backspan portion by pressing-the end of the backspan into an end of the leg portion opposite the pointed tip while the leg is in a molten state. The bond between the leg portions and the backspan portion can be an adhesive one that results from the cooling of the polymer in contact with metal. Part of the adhesion may result from shrinkage of the polymer upon cooling when it is in a configuration so as to surround the metal backspan portion. Other conventional methods such as casting from a solvent or melt pressing can also be used to join the leg to the backspan. Alternatively, shape memory polymeric legs may be inserted into hollow metal tubes at the ends of the backspan; this may be especially useful where the backspan portion is curved at its ends to permit placement of the shape memory leg into the upper leg portion found on the backspan. In such a case, a bioabsorbable adhesive can be useful for adhering the shape memory polymeric legs to the backspan portion.

The legs may possess their permanent shape prior to their attachment to the backspan or, in other embodiments, the legs may be formed so that they possess their permanent shape as they are attached to the backspan or, in other embodiments, the legs may be treated to possess their permanent shape after their attachment to the backspan. Similarly, the legs may be deformed to their temporary shape prior to their attachment to the backspan or, in other embodiments, the legs may be deformed to their temporary shape after their attachment to the backspan.

The implantation of a staple of the present disclosure in its temporary shape, and the transition thereof to its permanent shape in vivo, will now be described. The staple can be dispensed from a conventional mechanical device into the skin to close a break in the skin such as a wound, i.e., the staple may be dispensed into the skin adjacent a wound and become affixed to the tissue, thereby closing the wound. Staples of the present disclosure having the shape memory legs described herein may have a temporary shape prior to implantation wherein the legs are straight; thus a staple 10 having such legs may possess a standard U shape configuration as depicted in FIG. 5.

Upon implantation in the body, the heat of the body (about 37° C) may result in the staple 10 with legs having the straight, temporary shape depicted in FIG. 5 to return to its permanent shape having legs with a bent configuration that will permit the staple to grab and adhere to tissue, thereby retaining the staple in tissue and closing the wound to which they are applied. A staple 10 of the present disclosure having legs formed of a shape-memory polymer as-described herein that has returned to its permanent bent or curved shape may have a closed configuration as depicted in FIG. 4. In other embodiments, heat may be applied to the staples, in embodiments from about 39° C to about 43° C (just above human body temperature), to enhance the return of the shape memory polymer to its permanent bent or curved shape so that the staple 10 has a closed configuration as depicted in FIG. 4.

Again referring to FIG. 2, after sufficient healing has taken place, backspan 12 will have separated from the bioabsorbable shape memory legs 14 and 16 of staple 10 due to the degradation of legs 14 and 16 in vivo. Thus, backspan 12 may be removed simply by application of gentle traction on the exposed backspan portion 12 by, for example, plucking the metal using-fingemails or tweezers, or by the action of a wash cloth used during bathing. An alternative method for rapid staple removal is to place a -strip of adhesive tape on top of a row of staples utilized to close a wound in the skin and then to quickly strip off the tape which will remove all the backspan portions 12 of the staples.

The bioabsorbable shape memory legs 14 and 16, which are not necessarily completely absorbed at this time, remain buried under the skin and are slowly absorbed by the body. As noted above, times for absorption can range from about 5 days to about 2 years, in embodiments from about 1 week to about 3 months, depending on the composition of the leg portions and their shapes. Any shape memory polymer utilized to form legs 14 and 16 that remains implanted should have no effect-on the rapid healing-of any holes made by the staple 10 upon attachment to tissue.

In embodiments, a variety of optional ingredients including medicinal agents may be combined with the-shape-memory polymers utilized to form the legs of staples of the present disclosure. The term "medicinal agent", as used herein, is used in its broadest sense and includes any substance or mixture of substances that have clinical use.
Consequently, medicinal agents may-or may not have pharmacological activity per se, e.g., a dye. Alternatively a medicinal agent could be any agent which provides a therapeutic or prophylactic effect, a compound that affects or participates in tissue growth, cell growth, and/or cell differentiation, a compound that may be able to invoke a biological action such as an immune response, or a compound that could play any other role in one or more biological processes.

Examples of classes of medicinal agents which may be utilized in accordance with the present disclosure include antimicrobials; analgesics; antipyretics; anesthetics; antiepileptics; antihistamines; anti-inflammatory agents such as hormonal agents, hydrocortisone, prednisolone, prednisone, non hormonal agents, allopurinol, indomethacin, phenylbutazone and the like; cardiovascular agents such as coronary vasodilators and nitroglycerin; diagnostic agents; sympathomimetics; cholinomimetics; antimuscarinics; antispasmodics; hemostats to halt or prevent bleeding; muscle relaxants; adrenergic neuron blockers; antineoplastics; immunogenic agents; immunosuppressants; gastrointestinal drugs; diuretics; steroids; lipids; lipopolysaccharides; polysaccharides; and enzymes. It is also intended that combinations of medicinal agents may be used.

Other medicinal agents which may be included with the shape memory polymers utilized to form the legs of staples of the present disclosure include local anesthetics; non-steroidal antifertility agents; parasympathomimetic agents; psychotherapeutic agents; tranquilizers; sedative hypnotics; sulfonamides; vaccines; vitamins; antimalarials; anti-migraine agents; anti-parkinson agents-such as L-dopa; anti-spasmodics; anticholinergic agents (e.g. oxybutynin); bronchodilators; alkaloids; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anticoagulants; anti-convulsants; antidepressants; anti-emetics; prostaglandins and cytotoxic drugs; estrogens; antibiotics; anti-fungals; anti-virals; and immunological agents.

Suitable antimicrobial agents which may be included as a medicinal agent with the shape memory polymers utilized to form the legs of staples-of the present disclosure include triclosan, also known as 2,4,4'-trichloro-2'-hydroxydiphenyl ether, chlorhexidine and its-salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a medicinal agent with the shape memory polymers utilized to form the legs of staples of the present disclosure.

Examples of hemostat materials which can be employed include fibrin-based, collagen-based oxidized regenerated cellulose-based, and gelatin-based topical hemostats. Examples of commercially available hemostat materials include fibrinogen-thrombin combination materials sold under the trade designations COSTASIS^{™} by Tyco Healthcare Group, LP, and TISSEEL^{™} sold by Baxter International, Inc. Hemostats herein also include astringents, for example, aluminum sulfate, and coagulants.

Other examples of suitable medicinal agents which may be included with the shape memory polymers utilized to form the legs of staples of the present disclosure include viruses and cells, peptides, polypeptides and proteins, analogs, muteins, and active fragments thereof, such as immunoglobulins, antibodies, cytokines (e.g. lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-cancer and/or anti-tumor agents and tumor suppressors, -blood proteins, gonadotropins (e:g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic acids, such as antisense molecules, DNA and RNA; oligonucleotides; and ribozymes.

A single medicinal agent may be utilized with the-shape memory polymers utilized to form the legs of staples of the present disclosure or, in alternate embodiments, any combination of medicinal agents may be utilized with the shape memory polymers utilized to form the legs of staples of the present disclosure.

The medicinal agent may be disposed on a surface of a leg of a staple of the present disclosure or impregnated in or combined with the shape memory polymers utilized to form the legs of staples of the present disclosure.

Additionally, an enzyme may be added to the shape memory polymers utilized to form the legs of staples of the present disclosure to increase their rate of degradation. Suitable enzymes include, for example, peptide hydrolases such as elastase, cathepsin G, cathepsin E, cathepsin B, cathepsin H, cathepsin L, trypsin, pepsin, chymotrypsin, γ-glutamyltransferase (γ-GTP) and the like; -sugar chain hydrolases such as phosphorylase, neuraminidase, dextranase, amylase, lysozyme, oligosaccharase and the like; oligonucleotide hydrolases such as alkaline phosphatase, endoribonuclease, endodeoxyribonuclease and the like. In some embodiments, where an enzyme is added, the enzyme may be included in a liposome or microsphere to control the rate of its release, thereby controlling the rate of degradation of the legs of a staple of the present disclosure. Methods for incorporating enzymes into liposomes and/or microspheres are within the purview of those skilled in the art.

Methods for closing a wound with staples of the present disclosure are also provided. In embodiments, a wound, such as an incision, may be closed with a row of staples of the present disclosure dispensed by any means within the purview of one skilled in the art, including conventional mechanical devices. The number of staples necessary to close a wound may vary depending upon the configuration and length of the wound to be closed.

Staples of the present disclosure may possess certain advantages compared with skin staples known in the art. They require no significant modification of either insertion hardware or user procedures for insertion; the load bearing strength of the metallic central backspan portion is maintained; staple removal is required only for the central backspan portion; the pain of removal is minimized since the bioabsorbable legs remain embedded; and the cost of staple removal can be eliminated since it can be carried out by the patient rather than by medical-personnel.

While the above description contains many specifics, these specifics should not be construed as limitations on the scope of the disclosure, but-merely as exemplifications of embodiments thereof. Those skilled in the art will envision many other possibilities within the scope of the claims appended hereto.

## Claims

1. A surgical staple (10) comprising:
a backspan (2) comprising a non-absorbable materials; and
two leg (4, 6) at least a portion of each of the two legs comprising a bioabsorbable thermal shape memory polymeric materials, **characterised in that** the said leg portions have been deformed from a memorised bent or curved shape to a temporary insertion shape at a deforming temperature above the transition temperature (Tₜᵣₐₙₛ) but below the permanent temperature (Tₚₑᵣₘ) of the thermal shape memory polymeric material.

2. The staple of claim 1, wherein the non-absorbable material comprises a metal selected from the group consisting of titanium, stainless steel, steel alloys, titanium alloys, nickel chromium alloy, nickel/cobalt/chrome, and combinations thereof.

3. The staple of claim 1, wherein the non-absorbable material comprises titanium.

4. The staple of claim 1, wherein the shape memory polymer comprises a copolymer of a diol ester and an ether-ester diol:

5. The staple of claim 1, wherein the shape memory polymer is selected from the group consisting of oligo (epsilon caprolactone) diol/oligo (p-dioxanone) diol copolymers, lactide, glycolide, and combinations thereof.

6. The staple of claim 1, wherein the shape memory polymer comprises a blend of materials selected from the group consisting of urethaues, lactic acid, glycolic acid, acrylates, caprolactones, homopolymers thereof, copolymers thereof, and combinations thereof.

7. The staple of claim 1, wherein the shape memory polymer comprises a blend selected from the group consisting of polylactic acid/urethane blends, polyglycolic acid/urethane blends, copolymers of lactic acid and glycolic acid/urethane blends, polycaprolactone dimethacrylate poly(butyl acrylate) blends, and combinations thereof.

8. The staple of claim 1, wherein the legs have a permanent memory comprising a bent shape and a temporary memory comprising a straight shape.

9. The staple of claim 1, wherein the backspan and the legs are joined by mechanical means selected from the group consisting of tongue and groove frictional coupling means, locking tapers, pins, screws, rivets, and combinations thereof.

10. The staple of claim 1, wherein the backspan and the legs are joined by means selected from the group consisting of injection molding, casting from a solvent, melt pressing, the use of bioabsorbable adhesives, and combinations thereof,

11. The staple of claim 1, wherein the legs further comprise a medicinal agent.

12. The staple of claim 11, wherein the medicinal agent is selected from the group consisting of antimicrobials, analgesics, antipyretics, anesthetics, antiepileptics, antihistamines, anti-inflammatory agents, cardiovascular agents, diagnostic agents, sympathomimetics, cholinomimetics, antimuscarinics, antispasmodics, hemostats, muscle relaxants, adrenergic neuron blockers, antineoplastics, immunogenic agents, immunosuppressants, gastrointestinal drugs, diuretics, steroids, lipids, lipopolysaccharides, polysaccharides, enzymes, local anesthetics, non-steroidal antifertility agents, parasympathomimetic agents, psychotherapeutic agents, tranquilizers, sedative hypnotics, sulfonamides, vaccines, vitamins, antimalarials, anti-migraine agents, auti-parkinson agents, anti-spasmodics, anticholinergic agents, bronchodilators, alkaloids, narcotics, non-narcotics, opioid receptor antagonists, anticoagulants, anti-convulsants, antidepressants, anti-emetics, prostaglandins, cytotoxic drugs, estrogens, antibiotics, anti-fungals, anti-virals, immunological agents, viruses, cells, peptides, polypeptides, proteins, muteins, vaccines, antigens, blood coagulation factors, growth factors, protein inhibitors, protein antagonists, protein agonists, nucleic acids, oligonucleotides, ribozymes, and combinations thereof

13. A staple according to claim 1, wherein the backspan comprises a non-absorbable material selected from the group consisting of titanium, stainless steel, steel alloys, titanium alloys, nickel chromium alloy, nickel/cobalt/chrome, and combinations thereof.

14. The staple of claim 13, wherein the shape memory polymer is selected from the group consisting of oligo (epsilon caprolactone) diol/oligo (p-dioxanone) diol copolymers, lactide, glycolide; and combinations thereof.

15. The staple of claim 13, wherein the shape memory polymer comprises a blend of materials selected from the group consisting of urethanes, lactic acid, glycolic acid, acrylates, caprolactones, homopolymers thereof, copolymers thereof, and combinations thereof.

16. The staple of claim 13, wherein the shape memory polymer comprises a blend selected from the group consisting of polylactic acid/urethane blends, polyglycolic acid/urethane blends, copolymers of lactic acid and glycolic acid/urethane blends, polycaprolactone dimethacrylate poly(butyl acrylate) blends, and combinations thereof.

17. The staple of claim 13, wherein the legs have a permanent memory comprising a bent shape and a temporary memory comprising a straight shape.

18. The staple of claim 13, wherein the legs further comprise a medicinal agent selected from the group consisting of antimicrobials, analgesics; antipyretics, anesthetics, antiepileptics, antihistamines, anti-inflammatory agents, cardiovascular agents, diagnostic, agents, sympathomimetics, cholinomimetics, antimuscarinics, antispasmodics, hemostats, muscle relaxants, adrenergic neuron blockers, antineoplastics, immunogenic agents, immunosuppressants, gastrointestinal drugs, diuretics, steroids, lipids, lipopolysaccharides, polysaccharides, enzymes, local anesthetics, non-steroidal antifertility agents, parasympathomimetic agents, psychotherapeutic agents, tranquilizers, sedative hypnotics, sulfonamides, vaccines, vitamins, antimalarials, anti-migraine agents, anti-parkinson agents, anti-spasmodics, anticholinergic agents, bronchodilators, alkaloids, narcotics, non-narcotics, opioid receptor antagonists, anticoagulants, anti-convulsants, antidepressants, anti-emetics, prostaglandins, cytotoxic drugs, estrogen, antibiotics, anti-fungals, anti-virals, immunological agents, viruses, cells, peptides, polypeptides, proteins, muteins, vaccines, antigens, blood coagulation factors, growth factors, protein inhibitors, protein antagonists, protein agonists, nucleic acids, oligonucleotides, ribozymes, and combinations thereof.

## Patentansprüche

1. Chirurgische Klammer (10), welche folgendes umfaßt:
einen Rückenbereich (2), welcher ein nicht-absorbierbares Material umfaßt, und
zwei Schenkel (4, 6), wobei wenigstens ein Teil jedes der beiden Schenkel ein biologisch absorbierbares Polymermaterial mit thermischem Formgedächtnis umfaßt, **dadurch gekennzeichnet, daß** die Schenkelabschnitte bei einer Verformungstemperatur, die oberhalb der Übergangstemperatur (Tₜᵣₐₙₛ), jedoch unterhalb der permanenten Temperatur (Tₚₑᵣₘ) des Polymermaterials mit thermischem Formgedächtnis liegt, von einer gespeicherten gebogenen oder gekrümmten Form zu einer temporären Einsetzform verformt wurden.

2. Klammer nach Anspruch 1, wobei das nicht-absorbierbare Material ein Metall umfaßt, welches aus der Gruppe ausgewählt ist, bestehend aus Titan, nichtrostendem Stahl, Stahllegierungen, Titaniegierungen, Nickel-Chrom-Legierung, Nickel/Kobalt/Chrom und Kombinationen davon.

3. Klammer nach Anspruch 1, wobei das nicht-absorbierbare Material Titan umfaßt.

4. Klammer nach Anspruch 1, wobei das Formgedächtnispolymer ein Copolymer eines Diolesters und eines Ether-Ester-D.iols umfaßt.

5. Klammer nach Anspruch 1, wobei das Formgedächtnispolymer aus der Gruppe ausgewählt ist, bestehend aus Oligo- (Epsilon-Caprolacton-) Diol/Oligo- (p-Dioxanon-) Diol-Copolymeren, Lactid, Glycolid und Kombinationen davon.

6. Klammer nach Anspruch 1, wobei das Formgedächtnispolymer ein Gemisch von Materialien umfaßt, die aus der Gruppe ausgewählt sind, bestehend aus Urethanen, Milchsäure, Glycolsäure, Acrylaten, Caprolactonen, Homopolymeren davon, Copolymeren davon und Kombinationen davon.

7. Klammer nach Anspruch 1, wobei das Formgedächtnispolymer ein Gemisch umfaßt, ausgewählt aus der Gruppe, bestehend aus Polymilchsäure/Urethan-Gemischen, Polyglycolsäure/Urethan-Gemischen, Copolymeren von Milchsäure und Glycolsäure/Urethan-Gemischen, Polycaprolactondimethacrylatpoly(butylacrylat)-Gemischen und Kombinationen davon.

8. Klammer nach Anspruch 1, wobei die Schenkel ein permanentes Formgedächtnis haben, welches eine gebogene Form umfaßt, und ein temporäres Formgedächtnis haben, welches eine gerade Form umfaßt.

9. Klammer nach Anspruch 1, wobei der Rückenbereich und die Schenkel durch mechanische Mittel miteinander verbunden sind, ausgewählt aus der Gruppe, bestehend aus Nut-und-Feder-Reibungskopplungsmitteln, Feststellkegeln, Stiften, Schrauben, Nieten und Kombinationen davon.

10. Klammer nach Anspruch 1, wobei der Rückenbereich und die Schenkel durch Mittel miteinander verbunden sind, die aus der Gruppe ausgewählt sind, bestehend aus Spritzgießen, Gießen aus einem Lösungsmittel, Schmelzpressen, Verwenden von biologisch absorbierbaren Klebemitteln und Kombinationen davon.

11. Klammer nach Anspruch 1, wobei die Schenkel weiterhin ein medizinisches Mittel umfassen.

12. Klammer nach Anspruch 11, wobei das medizinische Mittel aus der Gruppe ausgewählt ist, bestehend aus antimikrobiellen Mitteln, Analgetika, Antipyretika, Anästhetika, Antiepileptika, Antihistaminika, antiinflammatorischen Mitteln, kardiovaskulären Mitteln, diagnostischen Mitteln, Sympathomimetika, Cholinomimetika, Antimuscarinergika, Antispasmodika, Hämostatika, Muskelrelaxanzien, Antisympathikotonika, Antineoplastika, immunogenen Mitteln, Immunsuppressiva, gastrointestinalen Arzneimitteln, Diuretika, Steroiden, Lipiden, Lipopolysacchariden, Polysacchariden, Enzymen, Lokalanästhetika, nicht-steroidalen kontrazeptiven Mitteln, parasympathomimetischen Mitteln, psychotherapeutischen Mitteln, Tranquilizern, sedativen Hypnotika, Sulfonamiden, Impfstoffen, Vitaminen, Antimalariamitteln, Antimigränemitteln, Antiparkinsonmitteln, Antispasmodika, Anticholinergika, Bronchodilstatoren, Alkaloiden, Narkotika, Nicht-Narkotika, Opioidrezeptorantagonisten, Antikoagulanzien, Antikonvulsiva, Antidepressiva, Antiemetika, Prostaglandinen, zytotoxischen Arzneimitteln, Östrogenen, Antibiotika, Antipilzmitteln, antiviralen Mitteln, immunologischen Mitteln, Viren, Zellen, Peptiden, Polypeptiden, Proteinen, Muteinen, Impfstoffen, Antigenen, Blutgerinnungsfaktoren, Wachstumsfaktoren, Proteininhibitoren, Proteinantagonisten, Proteinagonisten, Nukleinsäuren, Oligonukleotiden, Ribozymen und Kombinationen davon.

13. Klammer nach Anspruch 1, wobei der Rückenbereich ein nicht-absorbierbares Material umfaßt, ausgewählt aus der Gruppe, bestehend aus Titan, nichtrostendem Stahl, Stahllegierungen, Titanlegierungen, Nickel-Chrom-Legierung, Nickel/Kobalt/Chrom und Kombinationen davon.

14. Klammer nach Anspruch 13, wobei das Formgedächtnispolymer aus der Gruppe ausgewählt ist, bestehend aus Oligo- (Epsilon-Caprolacton-) Diol/Oligo- (p-Dioxanon-) Diol-Copolymeren, Lactid, Glycolid und Kombinationen davon.

15. Klammer nach Anspruch 13, wobei das Formgedächtnispolymer ein Gemisch von Materialien umfaßt, die aus der Gruppe ausgewählt sind, bestehend aus Urethanen, Milchsäure, Glycolsäure, Acrylaten, Caprolactonen, Homopolymeren davon, Copolymeren davon und Kombinationen davon.

16. Klammer nach Anspruch 13, wobei das Formgedächtnispolymer ein Gemisch umfaßt, ausgewählt aus der Gruppe, bestehend aus Polymilchsäure/Urethan-Gemischen, Polyglycolsäure/Urethan-Gemischen, Copolymeren von Milchsäure und Glycolsäure/Urethan-Gemischen, Polycaprolactondimethacrylatpoly(butylacrylat)-Gemischen und Kombinationen davon.

17. Klammer nach Anspruch 13, wobei die Schenkel ein permanentes Formgedächtnis haben, welches eine gebogene Form umfaßt, und ein temporäres Formgedächtnis haben, welches eine gerade Form umfaßt.

18. Klammer nach Anspruch 13, wobei die Schenkel weiterhin ein medizinisches Mittel umfassen, ausgewählt aus der Gruppe, bestehend aus antimikrobiellen Mitteln, Analgetika. Antipyretika, Anästhetika, Antiepileptika, Antihistaminika, antiinflammatorischen Mitteln, kardiovaskulären Mitteln, diagnostischen Mitteln, Sympathomimetika, Cholinomimetika, Antimuscarinergika. Antispasmodika, Hämostatika, Muskelrelaxanzien, Antisympathikotonika, Antineoplastika, immunogenen Mitteln, lmmunsuppressiva, gastrointestinalen Arzneimitteln, Diuretika, Steroiden, Lipiden, Lipopolysacchariden, Polysacchariden, Enzymen, Lokalanästhetika, nicht-steroidalen kontrazeptiven Mitteln, parasympathomimetischen Mitteln, psychotherapeutischen Mitteln, Tranquilizern, sedativen Hypnotika, Sulfonamiden, Impfstoffen, Vitaminen, Antimalariamitteln, Antimigränemitteln, Antiparkinsonmitteln, Antispasmodika, Anticholinergika, Bronchodilatatoren, Alkaloiden, Narkotika, Nicht-Narkotika, Opioidrezeptorantagonisten, Antikoagulanzien, Antikonvulsiva, Antidepressiva, Antiemetika, Prostaglandinen, zytotoxischen Arzneimitteln, Östrogenen, Antibiotika, Antipilzmitteln, antiviralen Mitteln, immunologischen Mitteln, Viren, Zellen, Peptiden, Polypeptiden, Proteinen, Muteinen, Impfstoffen, Antigenen, Blutgerinnungsfaktoren, Wachstumsfaktoren, Proteininhibitoren, Proteinantagonisten, Proteinagonisten, Nukleinsäuren, Oligonukleotiden, Ribozymen und Kombinationen davon.

## Revendications

1. Agrafe chirurgicale (10), comprenant :
une âme transversale (2), comprenant une matière non résorbable ; et
deux branches (4, 6), au moins une partie de chacune des deux branches comprenant une matière polymère à mémoire de forme thermique biorésorbable, **caractérisée en ce que** lesdites portions des branches ont été déformées d'une forme fléchie ou incurvée mémorisée à une forme d'insertion temporaire à une température de déformation supérieure à la température de transition (Tₜᵣₐₙₛ) mais inférieure à la température permanente (Tₚₑᵣₘ) de la matière polymère à mémoire de forme thermique.

2. Agrafe suivant la revendication 1, dans laquelle la matière non résorbable comprend un métal choisi dans le groupe consistant en le titane, l'acier inoxydable, des alliages d'acier, des alliages de titane, un alliage nickel-chrome, le nickel/cobalt/chrome et leurs associations.

3. Agrafe suivant la revendication 1, dans laquelle la matière non résorbable comprend le titane.

4. Agrafe suivant la revendication 1, dans laquelle le polymère à mémoire de forme comprend un copolymère d'un ester-diol et d'un éther-ester-diol.

5. Agrafe suivant la revendication 1, dans laquelle le polymère à mémoire de forme est choisi dans le groupe consistant en des copolymères oligo(epsilon-caprolactone)-diol/oligo(p-dioxannone)diol, un lactide, un glycolide et leurs associations.

6. Agrafe suivant la revendication 1, dans laquelle le polymère à mémoire de forme comprend un mélange de matières choisies dans le groupe consistant en des uréthannes, l'acide lactique, l'acide glycolique, des acrylates, des caprolactones, leurs homopolymères, leurs copolymères et leurs associations.

7. Agrafe suivant la revendication 1, dans laquelle le polymère à mémoire de forme comprend un mélange choisi dans le groupe consistant en des mélanges acide polylactique/ uréthanne, des mélanges acide polyglycolique/uréthanne, des mélanges copolymères d'acide lactique et d'acide glycolique/ uréthanne, des mélanges polycaprolactone-diméthacrylate/poly-(acrylate de butyle) et leurs associations.

8. Agrafe suivant la revendication 1, dans laquelle les branches ont une mémoire permanente comprenant une forme fléchie et une mémoire temporaire comprenant une forme droite.

9. Agrafe suivant la revendication 1, dans laquelle l'âme transversale et les branches sont jointes par un moyen mécanique choisi dans le groupe consistant en un moyen de couplage par frottement à languette et rainure, un raccord conique de verrouillage, des broches, des vis, des rivets et leurs associations.

10. Agrafe suivant la revendication 1, dans laquelle l'âme transversale et les branches sont jointes par un moyen mécanique choisi dans le groupe consistant en le moulage par injection, la coulée au solvant, la compression en masse fondue, l'utilisation d'adhésifs biorésorbables et leurs associations.

11. Agrafe suivant la revendication 1, dans laquelle les branches comprennent en outre un agent médicamenteux.

12. Agrafe suivant la revendication 11, dans laquelle l'agent médicamenteux est choisi dans le groupe consistant en les agents antimicrobiens, les analgésiques, les antipyrétiques, les anesthésiques, les antiépileptiques, les antihistaminiques, les agents anti-inflammatoires, les agents cardiovasculaires, les agents de diagnostic, les agents sympathomimétiques, les agents cholinomimétiques, les antimuscariniques, les antispasmodiques, les agents hémostatiques, les myorelaxants, les agents de blocage des neurones adrénergiques, les agents antinéoplasiques, les agents immunogènes, les agents immunosuppresseurs, les médicaments gastro-intestinaux, les diurétiques, les stéroïdes, les lipides, les lipopolysaccharides, les polysaccharides, les enzymes, les anesthésiques locaux, les agents contraceptifs non stéroïdiens, les agents parasympathomimétiques, les agents psychothérapeutiques, les tranquillisants, les hypnotiques sédatifs, les sulfonamides, les vaccins, les vitamines, les agents antipaludiques, les agents antimigraineux, les antiparkinsoniens, les antispasmodiques, les agents anticholinergiques, les branchodilatateurs, les alcaloïdes, les narcotiques, les non-narcotiques, les antagonistes des récepteurs d'opioïdes, les anticoagulants, les anticonvulsivants, les antidépresseurs, les antiémétiques, les prostaglandines, les médicaments cytotoxiques, les oestrogènes, les antibiotiques, les agents antifongiques, les agents antiviraux, les agents immunologiques, les virus, les cellules, les peptides, les polypeptides, les protéines, les mutéines, les vaccins, les antigènes, les facteurs de la coagulation sanguine, les facteurs de croissance, les inhibiteurs de protéines, les antagonistes de protéines, les agonistes de protéines, les acides nucléiques, les oligonucléotides, les ribozymes et leurs associations.

13. Agrafe suivant la revendication 1, dans laquelle l'âme transversale comprend une matière non résorbable choisie dans le groupe consistant en le titane, l'acier inoxydable, des alliages d'acier, des alliages de titane, un alliage nickel-chrome, le nickel/cobalt/chrome et leurs associations.

14. Agrafe suivant la revendication 13, dans laquelle le polymère à mémoire de forme est choisi dans le groupe consistant en des copolymères oligo(epsilon-caprolactone)-diol/oligo(p-dioxannone)diol, un lactide, un glycolide et leurs associations.

15. Agrafe suivant la revendication 13, dans laquelle le polymère à mémoire de forme comprend un mélange de matières choisi dans le groupe consistant en des uréthannes, l'acide lactique, l'acide glycolique, des acrylates, des caprolactones, leurs homopolymères, leurs copolymères et leurs associations.

16. Agrafe suivant la revendication 13, dans laquelle le polymère à mémoire de forme comprend un mélange choisi dans le groupe consistant en des mélanges acide polylactique/ uréthanne, des mélanges acide polyglycolique/uréthanne, des mélanges de copolymères d'acide lactique et d'acide glycolique/ uréthanne, des mélanges polycaprolactone-diméthacrylate/poly-(acrylate de butyle) et leurs associations.

17. Agrafe suivant la revendication 13, dans laquelle les branches ont une mémoire permanente comprenant une forme fléchie et une mémoire temporaire comprenant une forme droite.

18. Agrafe suivant la revendication 13, dans laquelle les branches comprennent en outre un agent médicamenteux choisi dans le groupe consistant en les agents antimicrobiens, les analgésiques, les antipyrétiques, les anesthésiques, les antiépileptiques, les antihistaminiques, les agents anti-inflammatoires, les agents cardiovasculaires, les agents de diagnostic, les agents sympathomimétiques, les agents cholinomimétiques, les antimuscariniques, les antispasmodiques, les agents hémostatiques, les myorelaxants, les agents de blocage des neurones adrénergiques, les agents antinéoplasiques, les agents immunogènes, les agents immunosuppresseurs, les médicaments gastro-intestinaux, les diurétiques, les stéroïdes, les lipides, les lipopolysaccharides, les polysaccharides, les enzymes, les anesthésiques locaux, les agents contraceptifs non stéroïdiens, les agents parasympathomimétiques, les agents psychothérapeutiques, les tranquillisants, les hypnotiques sédatifs, les sulfonamides, les vaccins, les vitamines, les agents antipaludiques, les agents antimigraineux, les antiparkinsoniens, les antispasmodiques, les agents anticholinergiques, les bronchodilatateurs, les alcaloïdes, les narcotiques, les non-narcotiques, les antagonistes des récepteurs d'opioïdes, les anticoagulants, les anticonvulsivants, les antidépresseurs, les antiémétiques, les prostaglandines, les médicaments cytotoxiques, les oestrogènes, les antibiotiques, les agents antifongiques, les agents antiviraux, les agents immunologiques, les virus, les cellules, les peptides, les polypeptides, les protéines, les mutéines, les vaccins, les antigènes, les facteurs de la coagulation sanguine, les facteurs de croissance, les inhibiteurs de protéines, les antagonistes de protéines, les agonistes de protéines, les acides nucléiques, les oligonucléotides, les ribozymes et leurs associations.
